# FASCICULE DE BREVET EUROPEEN

(11) **EP 4 096 573 B1**
(45) Date de publication et mention de la délivrance du brevet: **02.09.2026**
(21) Numéro de dépôt: 21707321.2
(22) Date de dépôt: 25.01.2021
(51) Int. Cl.: A61D 7/00, A61M 11/00, A61M 11/02

(54) **DISPOSITIF DE DISTRIBUTION DE PRODUIT PULVERULENT**
VORRICHTUNG ZUR AUSGABE EINES PULVERFÖRMIGEN PRODUKTS
DEVICE FOR DISPENSING A PULVERULENT PRODUCT

(30) Priorité: 27.01.2020 FR 2000780
(43) Date de publication de la demande: 07.12.2022
(73) Titulaire: APTAR France SAS, 27110 Le Neubourg (FR)
(72) Inventeur: LE MANER, François, 27400 La Vallee Montaure (FR)
(74) Mandataire: CAPRI
(86) Numéro de dépôt international: PCT/FR2021/050127
(87) Numéro de publication internationale: WO 2021/152249

(56) Documents cités:
- WO-A1-2009/029028
- WO-A1-2019/073165

## Description

La présente invention concerne un dispositif de distribution de produit pulvérulent, notamment destiné à l'administration de produit pharmaceutique sous forme de poudre à des petits animaux, tels que par exemple des rongeurs, notamment des souris.

Les dispositifs de l'état de la technique utilisés pour administrer des doses de poudre à des petits animaux, tels que des rongeurs, notamment des souris, comportent généralement un réservoir contenant une seule dose de poudre, associé à une tête de distribution d'une part, et à un système de chasse d'air d'autre part. Lors de l'actionnement, la chasse d'air génère un écoulement d'air sous pression qui permet d'expulser la dose de poudre à travers la tête de distribution, puis généralement à travers une canule intubée dans l'animal pour distribuer la poudre au niveau de sa carène trachéale.

Ces dispositifs présentent généralement des inconvénients. Ainsi, après chaque actionnement, il faut remplacer le réservoir vide par un réservoir plein, ce qui n'est ni commode pour le manipulateur ni économique. Une solution pour résoudre ce problème est de prévoir un réservoir réutilisable, par exemple pouvant aisément être rempli avec une nouvelle dose de poudre et assemblé dans le dispositif avant chaque actionnement.

Un autre inconvénient concerne la chasse d'air, généralement réalisée par une seringue contenant de l'air. Avec ce type de chasse d'air, l'écoulement d'air sous pression généré lors de l'actionnement est dépendant de la manière dont l'utilisateur actionne le dispositif, notamment de la force avec laquelle il réalise son actionnement. Ceci ne permet pas de réaliser une distribution reproductible à chaque actionnement. Une solution pour résoudre ce problème est d'utiliser une pompe adaptée à générer un écoulement d'air sous pression, l'actionnement de cette pompe étant indépendant de la force exercée par l'utilisateur, notamment de la vitesse à laquelle il réalise cet actionnement.

Les documents WO2019073165 et WO2009029028 décrivent des dispositifs de l'état de la technique.

La présente invention a pour but de fournir un dispositif de distribution de poudre qui ne reproduit pas les inconvénients susmentionnés.

La présente invention a notamment pour but de fournir un dispositif de distribution de poudre qui permette de distribuer plusieurs doses en plusieurs actionnements successifs.

La présente invention a également pour but de fournir un dispositif de distribution de poudre qui permette de remplir aisément le réservoir avec une dose de poudre avant chaque actionnement.

La présente invention a aussi pour but de fournir un dispositif de distribution de poudre qui soit simple et fiable d'utilisation, avec une distribution reproductible à chaque actionnement.

La présente invention a également pour but de fournir un dispositif de distribution de poudre qui soit simple et peu coûteux à fabriquer et à assembler.

La présente invention a donc pour objet un dispositif de distribution de produit fluide comportant une unité réservoir reliée d'une part à un système de chasse d'air et d'autre part à une tête de distribution pourvue d'un orifice de distribution, ledit système de chasse d'air comportant une pompe comportant un corps externe qui contient un corps de pompe comportant une chambre de pompe et dans lequel est disposée une tige formant piston, ladite tige étant fixe par rapport audit corps externe, et ledit corps de pompe étant déplaçable axialement pour coulisser autour de ladite tige entre une position de repos et un position d'actionnement, ledit corps de pompe comportant un corps creux proximal par rapport audit orifice de distribution et un corps creux distal par rapport audit orifice de distribution fixés l'un à l'autre, ledit corps creux proximal comportant une partie de corps de pompe proximale par rapport audit orifice de distribution et une partie de corps de pompe distale par rapport audit orifice de distribution dont le diamètre intérieur est supérieur à celui de ladite partie de corps de pompe proximale, ladite tige comportant une partie de tige proximale par rapport audit orifice de distribution, une partie de tige centrale et une partie de tige distale par rapport audit orifice de distribution, le diamètre extérieur de ladite partie de tige centrale étant supérieur aux diamètres desdites parties de tige proximale et distale, ladite partie de tige centrale supportant un premier joint et un second joint, et ladite partie de tige distale supportant un troisième joint, ladite partie de tige proximale comportant un canal central s'étendant jusque dans ladite partie de tige centrale au-delà dudit premier joint et qui débouche latéralement à l'extérieur de ladite partie de tige centrale entre lesdits premier et second joints, ledit corps creux distal contenant au moins un ressort coopérant d'une part avec ladite partie de tige distale et d'autre part avec ledit corps creux distal.

Avantageusement, ledit corps creux proximal et ledit corps creux distal sont fixés l'un à l'autre, notamment par vissage, de manière étanche avec interposition d'un joint de corps.

Avantageusement, ladite partie de tige distale coulisse dans ledit corps creux distal, ledit troisième joint réalisant l'étanchéité entre eux en cours d'actionnement.

Avantageusement, ledit corps creux distal contient deux ressorts disposés axialement l'un derrière l'autre en étant relié par un organe de liaison.

Avantageusement, un élément de réglage, tel qu'une vis de réglage, est disposé dans le corps creux distal pour former le contact avec ledit ressort, permettant de modifier la force d'actionnement exercée par le ou les ressort(s) sur ladite tige lors de l'actionnement.

Avantageusement, ladite unité réservoir comporte un réservoir ayant sensiblement la forme d'un cylindre creux, avec une ouverture distale, une ouverture proximale et un canal de dosage reliant lesdites ouvertures distale et proximale, un clapet unidirectionnel étant disposé entre ledit canal de dosage et ladite ouverture distale, ladite ouverture proximale dudit réservoir formant un cône de remplissage pour faciliter le remplissage dudit canal de dosage avec une dose de poudre.

Avantageusement, ledit clapet unidirectionnel est du type à membrane fendue qui ne s'ouvre que dans une seule direction sous l'effet d'une pression prédéterminée.

Avantageusement, ladite tête de distribution comporte un organe de distribution pourvu dudit orifice de distribution.

Avantageusement, ledit organe de distribution comporte une aiguille.

Avantageusement, ladite aiguille est courbe ou coudée.

Ces caractéristiques et avantages et d'autres de la présente invention apparaîtront plus clairement au cours de la description détaillée suivante, faite en référence aux dessins joints, donnés à titre d'exemples non limitatifs, et sur lesquels :
la figure 1 est une vue schématique en perspective découpée d'un dispositif de distribution de poudre selon un mode de réalisation avantageux, en position avant actionnement,
la figure 2 est une vue similaire à celle de la figure 1, après actionnement,
la figure 3 est une vue en perspective éclatée d'un système de chasse d'air selon un mode de réalisation avantageux,
la figure 4 est une vue en perspective de côté du système de chasse d'air de la figure 3,
les figures 5 à 10 sont des vues en perspective découpée du système de chasse d'air de la figure 3, montrant les différentes étapes d'un cycle d'actionnement dudit système de chasse d'air,
la figure 11 est une vue agrandie du détail D de la figure 6,
la figure 12 est une vue en perspective découpée d'un ensemble réservoir et tête de distribution, selon un mode de réalisation avantageux, et
la figure 13 une vue de détail du réservoir de la figure 12.

Les termes "proximal" et "distal" se réfèrent à l'orifice de distribution. Les termes "amont" et "aval" se réfèrent à la direction d'écoulement du produit fluide lors de sa distribution. Les termes "axial" et "radial" se réfèrent à l'axe central longitudinal du dispositif.

Le dispositif de distribution de produit fluide représenté sur les figures comporte une unité réservoir 100 pouvant contenir une dose de produit pulvérulent, relié d'un côté à un système de chasse d'air 200 et de l'autre côté à une tête de distribution 300 pourvue d'un orifice de distribution 310.

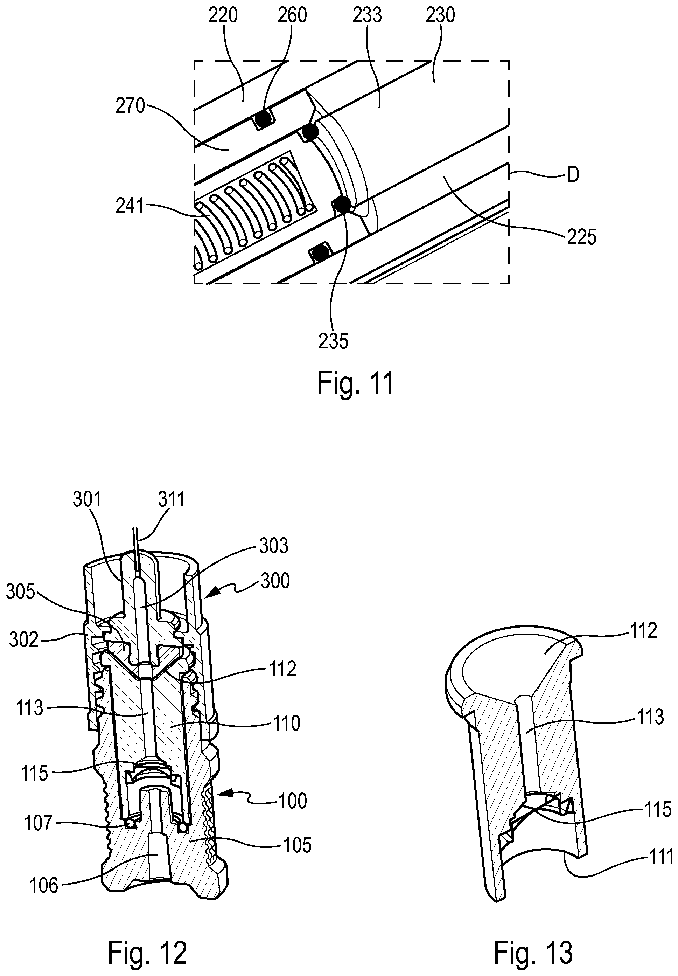

L'unité réservoir 100, plus particulièrement visible sur les figures 12 et 13, comporte un réservoir 110 ayant sensiblement la forme d'un cylindre creux, avec une ouverture distale 111, une ouverture proximale 112 et un canal de dosage 113 reliant lesdites deux ouvertures distale et proximale 111, 112. Un clapet unidirectionnel 115 est disposé entre ledit canal de dosage 113 et ladite ouverture distale 111.

Ainsi, lorsque le réservoir 110 est rempli avec une dose de poudre à travers l'ouverture proximale 112, cette dose de poudre reste dans le canal de dosage 113, le clapet unidirectionnel 115 l'empêchant de sortir par l'ouverture distale 111. Lorsque le système de chasse d'air 200 est actionné, un écoulement d'air sous pression est envoyé dans le réservoir 110 à travers ladite ouverture distale 111, provoquant l'ouverture du clapet unidirectionnel 115 et l'expulsion de la dose de poudre à travers l'ouverture proximale 112.

Le réservoir 110 est de préférence fixé dans un corps cylindrique 105 comportant un canal central 106 permettant de relier le système de chasse d'air 200 avec le réservoir 110.

Comme visible sur la figure 13, l'ouverture proximale 112 du réservoir 110 forme un cône de remplissage pour faciliter le remplissage du canal de dosage 113 avec la dose de poudre. En effet, les doses destinées aux petits animaux, tels que les rongeurs, sont généralement très petites, typiquement de l'ordre de quelques mm³. Avantageusement, c'est le volume du canal de dosage 113 qui définit le volume de la dose de poudre distribuée à chaque actionnement. Cette forme tubulaire allongée du canal de dosage 113 permet notamment de limiter les risques d'agglomération de la poudre.

Le réservoir 110 est avantageusement fixé dans le corps cylindrique 105 avec interposition d'un joint d'étanchéité 107.

Le clapet unidirectionnel 115 peut être du type à membrane fendue qui ne s'ouvre que dans une seule direction sous l'effet d'une pression prédéterminée. D'autres mises en œuvre, par exemple une bille, sont possibles.

Une telle unité réservoir 100 permet notamment de réaliser aisément une stérilisation en autoclave.

Avantageusement, le réservoir 110 est réalisé en métal, notamment en acier inoxydable. Ceci permet d'éviter l'utilisation de matériaux plastiques dans le chemin d'expulsion de la poudre. Le corps cylindrique 105 peut aussi être réalisé en métal, notamment en acier inoxydable. De cette manière, l'unité réservoir 100 peut facilement être reliée à la terre pour éviter ou limiter les phénomènes d'électricité statique.

La tête de distribution 300 comporte un corps creux 301 fixé à l'unité réservoir 100 par une bague de fixation 302.

La bague de fixation 302 est fixée sur le corps cylindrique 105 de l'unité réservoir 100, par exemple par vissage, encliquetage ou sertissage. Tout autre moyen de fixation est possible.

Le corps creux 301 comporte un canal axial central 303, relié d'un côté à l'ouverture proximale 112 du réservoir 110 et de l'autre côté à un organe de distribution 311 pourvu d'un orifice de distribution 310. Dans l'exemple représenté, l'organe de distribution 311 comporte une aiguille, qui peut être courbe ou coudée. En variante, on pourrait envisager un organe de distribution permettant de délivrer un aérosol.

Avantageusement, un bouchon creux 305 est interposé entre le réservoir 110 et le corps creux 301, ledit bouchon creux 305 ayant une forme conique adaptée à la forme de l'ouverture proximale 112 du réservoir 110. Ce bouchon creux 305 permet d'assurer une bonne liaison entre le réservoir 110 et le corps creux 301, notamment une étanchéité suffisante pour éviter toute perte de dose lors de la distribution d'une dose de poudre.

Le système de chasse d'air 200 peut être de manière connue une seringue remplie d'air, l'actionnement du piston générant un écoulement d'air pressurisé.

De préférence toutefois, le système de chasse d'air 200 comporte une pompe dont l'actionnement est indépendant de la force d'actionnement exercée par l'utilisateur. Ainsi, l'écoulement d'air pressurisé généré à chaque actionnement est toujours identique et reproductible d'un actionnement à l'autre.

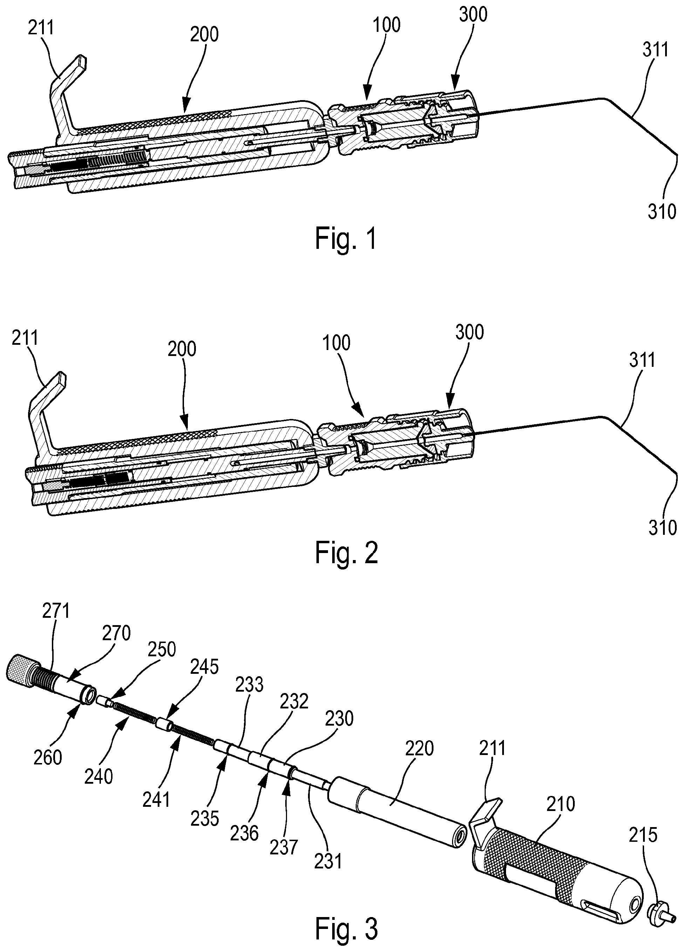

Un exemple avantageux du système de chasse d'air 200 va maintenant être décrit plus en détails en référence aux figures 1 à 11.

Dans cet exemple, la chasse d'air est formée par une pompe 200 comportant un corps externe 210 pourvu d'une poignée 211 et d'un embout de distribution 215. Le corps externe 210 contient un corps de pompe comportant une chambre de pompe 225 et dans lequel est disposée une tige 230 formant piston. La tige 230 est fixe par rapport au corps externe 210, et le corps de pompe est déplaçable axialement pour coulisser autour de ladite tige 230 entre une position de repos et un position d'actionnement.

Le corps de pompe comporte un corps creux proximal 220 et un corps creux distal 270, fixés l'un à l'autre, notamment par vissage. Un joint de corps 260 est prévu entre les corps creux proximal et distal pour assurer un fixation étanche de l'un sur l'autre. Le corps creux proximal 220 comporte une partie de corps de pompe proximale 221 et une partie de corps de pompe distale 222 dont le diamètre intérieur est supérieur à celui de ladite partie de corps de pompe proximale 221. Le corps creux distal 270 s'étend au moins partiellement dans le corps creux proximal 220.

Ladite tige 230 comporte une partie de tige proximale 231, une partie de tige centrale 232 et une partie de tige distale 233. Le diamètre extérieur de ladite partie de tige centrale 232 est supérieur aux diamètres des parties de tige proximale 231 et distale 233. La partie de tige centrale 232 supporte un premier joint 237 et un second joint 236, et la partie de tige distale 233 supporte un troisième joint 235. La partie de tige proximale 231 comporte un canal central 234 qui s'étend depuis l'embout de distribution 215 jusque dans la partie de tige centrale 232 au-delà dudit premier joint 237 et qui débouche latéralement à l'extérieur de ladite partie de tige centrale 232 entre lesdits premier et second joints 237, 236. La partie de tige distale 233 coulisse dans ledit corps creux distal 270, ledit troisième joint 235 réalisant l'étanchéité entre eux.

Le corps creux distal 270 contient au moins un ressort 240, 241 coopérant d'une part avec la partie de tige distale 233 et d'autre part avec le corps creux distal 270. Dans l'exemple représenté sur les dessins, il y a deux ressorts 240, 241 disposés axialement l'un derrière l'autre en étant relié par un organe de liaison 245. Bien entendu, un seul ressort est envisageable.

Eventuellement, un élément de réglage 250, tel qu'une vis de réglage, peut être disposé dans le corps creux distal 270 pour former le contact avec le ressort 240. Cet élément de réglage 250 permet alors de modifier aisément la force d'actionnement exercée par le ou les ressort(s) sur la tige 230 lors de l'actionnement.

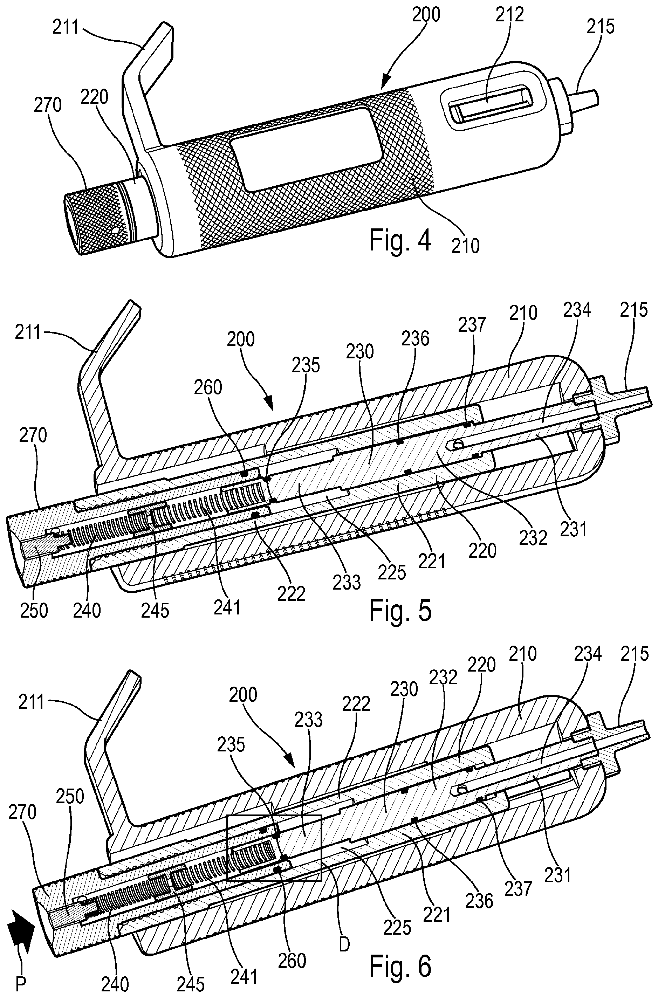

Les figures 5 à 10 illustrent un cycle d'actionnement de cette pompe à air avantageuse.

En position de repos, représentée sur la figure 5, le corps de pompe est sollicité axialement vers l'extérieur du corps externe 210 par les ressorts 240, 241, les premier et second joint 237, 236 coopèrent de manière étanche avec la partie de corps de pompe proximale 221 du corps creux proximal 220. La chambre de dosage 225 est donc isolée de l'embout de distribution. Le troisième joint 235 est disposé hors du corps creux distal 270, de sorte que la chambre de dosage 225 est ouverte sur l'atmosphère via le corps creux distal 270.

Lorsque l'utilisateur exerce une force de poussée axiale sur le corps creux distal 270, comme illustré par la flèche P sur la figure 6, le corps de pompe, formé par le corps creux proximal 220 et le corps creux distal 270, coulisse axialement vers l'intérieur du corps externe 210, autour de la tige 230. Comme mieux visible sur la figure 11, le troisième joint 235 vient alors coopérer des manière étanche avec la surface interne du corps creux distal 270, pour ainsi isoler la chambre de dosage 225 de l'atmosphère.

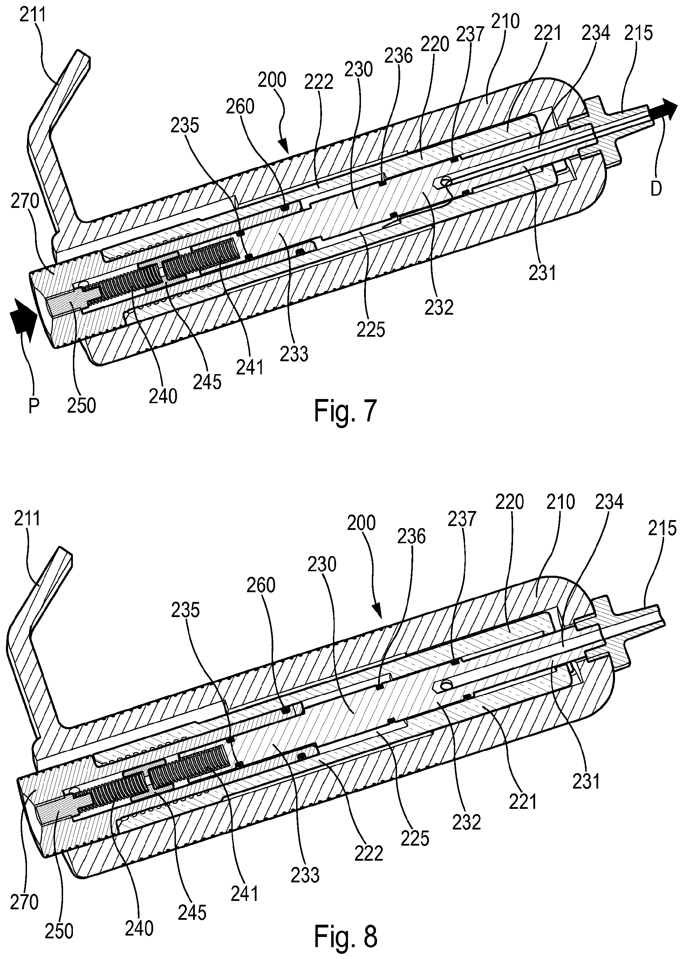

Une poursuite de la force de poussée axiale P telle qu'illustrée sur la figure 7 provoque alors la compression de l'air dans la chambre de dosage 225, sous l'effet de la partie de tige centrale 232 de plus grand diamètre qui pénètre progressivement dans la chambre de dosage 225. Lorsque le second joint 236 parvient dans la partie de corps de pompe distale 222 de plus grand diamètre et cesse de coopérer de manière étanche avec la partie de corps de pompe proximal 221, l'air comprimé contenu dans la chambre de dosage 225 peut s'échapper autour de la partie de tige centrale 232 jusqu'au canal central 234 puis à l'embout de distribution 215, comme illustré par la flèche D sur la figure 7.

La figure 8 montre la position en fin de course d'actionnement, après expulsion de l'air comprimé contenu dans la chambre de dosage 225. Dans cette position, l'extrémité axiale proximale du corps creux distal 270 vient en butée contre l'épaulement formé entre la partie de tige distale 233 et la partie de tige centrale 232. Cette butée mécanique de la course d'actionnement de la pompe assure un actionnement indépendant de la force exercée par l'utilisateur.

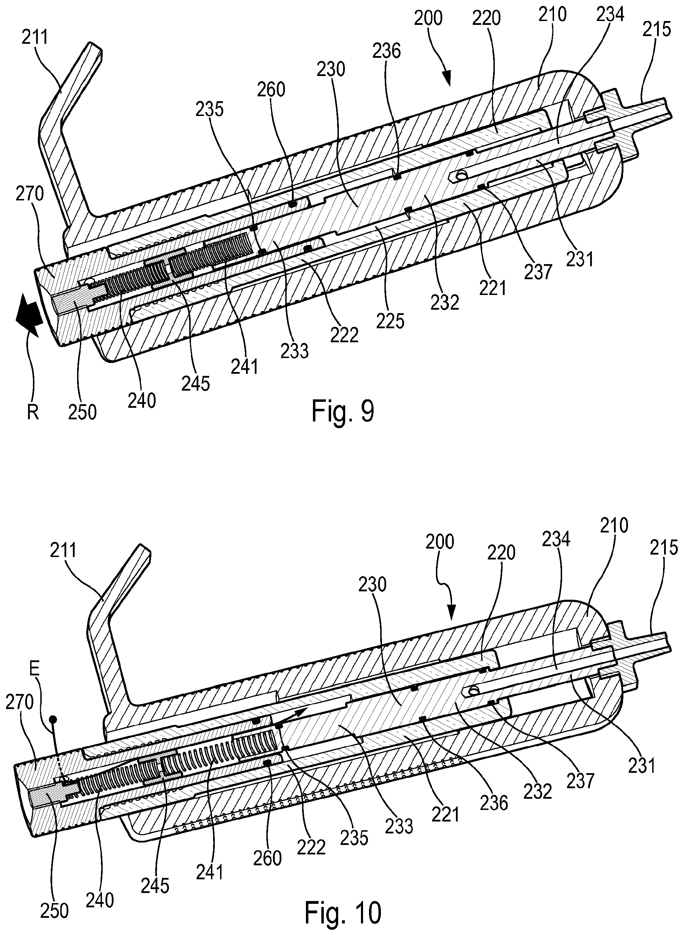

Lorsque l'utilisateur relâche sa pression sur le corps de pompe, le ressort 240, 241 qui a été comprimé lors de la course d'actionnement, se détend, ce qui ramène le corps de pompe vers sa position de repos, comme illustré par la flèche R sur la figure 9. Dès que le second joint 236 coopère à nouveau de manière étanche avec la partie de tige centrale 232, la chambre de dosage 225 est à nouveau isolée de l'embout de distribution 215, de sorte qu'il n'y a aucun risque de ré-aspiration de produit fluide ou d'air depuis l'animal traité, malgré la dépression qui se crée dans la chambre de dosage 225 lors de la course de retour.

Dès que le troisième joint 235 cesse sa coopération étanche avec la surface interne du corps creux distal 270, la chambre de dosage 225 s'ouvre à nouveau vers l'atmosphère générant un flux d'éventation à travers le corps creux distal 270, représenté par la flèche E dans la figure 10.

La pompe est alors prête pour une nouvelle utilisation.

Bien que la présente invention ait été décrite en référence à un mode de réalisation avantageux, il est entendu que l'homme du métier peut y apporter diverses modifications sans sortir du cadre de la présente invention tel que défini par les revendications annexées.

## Revendications

1. Dispositif de distribution de produit fluide comportant une unité réservoir (100) reliée d'une part à un système de chasse d'air (200) et d'autre part à une tête de distribution (300) pourvue d'un orifice de distribution (310), dans lequel ledit système de chasse d'air comporte une pompe (200) comportant un corps externe (210) qui contient un corps de pompe (220; 270) comportant une chambre de pompe (225) et dans lequel est disposée une tige (230) formant piston, ladite tige (230) étant fixe par rapport audit corps externe (210), et ledit corps de pompe (220; 270) étant déplaçable axialement pour coulisser autour de ladite tige (230) entre une position de repos et un position d'actionnement, ledit corps de pompe comportant un corps creux proximal (220) par rapport audit orifice de distribution et un corps creux distal (270) par rapport audit orifice de distribution fixés l'un à l'autre, ledit corps creux proximal (220) comportant une partie de corps de pompe proximale (221) par rapport audit orifice de distribution et une partie de corps de pompe distale (222) par rapport audit orifice de distribution dont le diamètre intérieur est supérieur à celui de ladite partie de corps de pompe proximale (221), ladite tige (230) comportant une partie de tige proximale (231) par rapport audit orifice de distribution, une partie de tige centrale (232) et une partie de tige distale (233) par rapport audit orifice de distribution, le diamètre extérieur de ladite partie de tige centrale (232) étant supérieur aux diamètres desdites parties de tige proximale (231) et distale (233), ladite partie de tige centrale (232) supportant un premier joint (237) et un second joint (236), qui, en position de repos, coopèrent de manière étanche avec ladite partie de corps de pompe proximale (221), et ladite partie de tige distale (233) supportant un troisième joint (235), qui, en cours d'actionnement, vient coopérer de manière étanche avec ledit corps creux distal (270) pour isoler ladite chambre de pompe (225) de l'atmosphère, l'air contenu dans ladite chambre de pompe (225) étant alors comprimé et ledit air comprimé étant libéré lorsque ledit second joint (236) ne coopère plus de manière étanche avec ladite partie de corps de pompe proximale (221), ladite partie de tige proximale (231) comportant un canal central (234) s'étendant jusque dans ladite partie de tige centrale (232) au-delà dudit premier joint (237) et qui débouche latéralement à l'extérieur de ladite partie de tige centrale (232) entre lesdits premier et second joints (237, 236), ledit corps creux distal (270) contenant au moins un ressort (240, 241) coopérant d'une part avec ladite partie de tige distale (233) et d'autre part avec ledit corps creux distal (270).

2. Dispositif selon la revendication 1, dans lequel ledit corps creux proximal (220) et ledit corps creux distal (270) sont fixés l'un à l'autre, notamment par vissage, de manière étanche avec interposition d'un joint de corps (260).

3. Dispositif selon la revendication 1 ou 2, dans lequel ladite partie de tige distale (233) coulisse dans ledit corps creux distal (270), ledit troisième joint (235) réalisant l'étanchéité entre eux en cours d'actionnement.

4. Dispositif selon l'une quelconque des revendications précédentes, dans lequel ledit corps creux distal (270) contient deux ressorts (240, 241) disposés axialement l'un derrière l'autre en étant relié par un organe de liaison (245).

5. Dispositif selon l'une quelconque des revendications précédentes, dans lequel un élément de réglage (250), tel qu'une vis de réglage, est disposé dans le corps creux distal (270) pour former le contact avec ledit ressort (240), permettant de modifier la force d'actionnement exercée par le ou les ressort(s) sur ladite tige (230) lors de l'actionnement.

6. Dispositif selon l'une quelconque des revendications précédentes, dans lequel ladite unité réservoir (100) comporte un réservoir (110) ayant sensiblement la forme d'un cylindre creux, avec une ouverture distale (111), une ouverture proximale (112) et un canal de dosage (113) reliant lesdites ouvertures distale et proximale (111, 112), un clapet unidirectionnel (115) étant disposé entre ledit canal de dosage (113) et ladite ouverture distale (111), ladite ouverture proximale (112) dudit réservoir (110) formant un cône de remplissage pour faciliter le remplissage dudit canal de dosage (113) avec une dose de poudre.

7. Dispositif selon la revendication 6, dans lequel ledit clapet unidirectionnel (115) est du type à membrane fendue qui ne s'ouvre que dans une seule direction sous l'effet d'une pression prédéterminée.

8. Dispositif selon l'une quelconque des revendications précédentes, dans lequel ladite tête de distribution (300) comporte un organe de distribution (311) pourvu dudit orifice de distribution (310).

9. Dispositif selon la revendication 8, dans lequel ledit organe de distribution (311) comporte une aiguille.

10. Dispositif selon la revendication 9, dans lequel ladite aiguille (311) est courbe ou coudée.

## Patentansprüche

1. Vorrichtung zur Ausgabe eines fluiden Produkts, die eine Behältereinheit (100) aufweist, die einerseits mit einem Entlüftungssystem (200) und andererseits mit einem Ausgabekopf (300) verbunden ist, der mit einer Ausgabeöffnung (310) versehen ist, wobei das Entlüftungssystem eine Pumpe (200) aufweist, die einen Außenkörper (210) aufweist, der einen Pumpenkörper (220; 270) enthält, der eine Pumpenkammer (225) aufweist und in dem eine Stange (230) angeordnet ist, die einen Kolben bildet, wobei die Stange (230) in Bezug auf den Außenkörper (210) feststehend ist und der Pumpenkörper (220; 270) axial verschiebbar ist, um um die Stange (230) zwischen einer Ruhestellung und einer Betätigungsstellung zu gleiten, wobei der Pumpenkörper einen in Bezug auf die Ausgabeöffnung proximalen Hohlkörper (220) und einen in Bezug auf die Ausgabeöffnung distalen Hohlkörper (270) aufweist, die miteinander verbunden sind, wobei der proximale Hohlkörper (220) einen in Bezug auf die Ausgabeöffnung proximalen Pumpenkörperabschnitt (221) und einen in Bezug auf die Ausgabeöffnung distalen Pumpenkörperabschnitt (222) aufweist, dessen Innendurchmesser größer als der des proximalen Pumpenkörperabschnitts (221) ist, wobei die Stange (230) einen in Bezug auf die Ausgabeöffnung proximalen Stangenabschnitt (231), einen mittleren Stangenabschnitt (232) und einen in Bezug auf die Ausgabeöffnung distalen Stangenabschnitt (233) aufweist, wobei der Außendurchmesser des mittleren Stangenabschnitts (232) größer als die Durchmesser des proximalen (231) und distalen (233) Stangenabschnitts ist, wobei der mittlere Stangenabschnitt (232) eine erste Dichtung (237) und eine zweite Dichtung (236) trägt, die in Ruhestellung dicht mit dem proximalen Pumpenkörperabschnitt (221) zusammenwirken, und der distale Stangenabschnitt (233) eine dritte Dichtung (235) trägt, die in Betätigung dicht mit dem distalen Hohlkörper (270) zusammenwirkt, um die Pumpenkammer (225) von der Atmosphäre zu isolieren, wobei die in der Pumpenkammer (225) enthaltene Luft dann komprimiert wird und die komprimierte Luft freigesetzt wird, wenn die zweite Dichtung (236) nicht mehr dicht mit dem proximalen Pumpenkörper (221) zusammenwirkt, wobei der proximale Stangenabschnitt (231) einen zentralen Kanal (234) aufweist, der sich bis in den mittleren Stangenabschnitt (232) über die erste Dichtung (237) hinaus erstreckt und der seitlich des mittleren Stangenabschnitts (232) zwischen der ersten und zweiten Dichtung (237, 236) nach außen mündet, wobei der distale Hohlkörper (270) mindestens eine Feder (240, 241) enthält, die einerseits mit dem distalen Stangenabschnitt (233) und andererseits mit dem distalen Hohlkörper (270) zusammenwirkt.

2. Vorrichtung nach Anspruch 1, wobei der proximale Hohlkörper (220) und der distale Hohlkörper (270) bei Zwischenlage einer Körperdichtung (260) insbesondere durch Verschraubung dichtend miteinander verbunden sind.

3. Vorrichtung nach Anspruch 1 oder 2, wobei der distale Stangenabschnitt (233) in dem distalen Hohlkörper (270) gleitet, wobei die dritte Dichtung (235) während der Betätigung die Dichtigkeit zwischen ihnen gewährleistet.

4. Vorrichtung nach einem der vorstehenden Ansprüche, wobei der distale Hohlkörper (270) zwei Federn (240, 241) enthält, die axial hintereinander angeordnet und durch ein Verbindungselement (245) miteinander verbunden sind.

5. Vorrichtung nach einem der vorstehenden Ansprüche, wobei ein Einstellelement (250), wie beispielsweise eine Einstellschraube, in dem distalen Hohlkörper (270) angeordnet ist, um den Kontakt mit der Feder (240) zu bilden, wodurch die von der oder den Federn bei der Betätigung auf die Stange (230) ausgeübte Betätigungskraft verändert werden kann.

6. Vorrichtung nach einem der vorstehenden Ansprüche, wobei die Behältereinheit (100) einen Behälter (110) aufweist, der im Wesentlichen die Form eines Hohlzylinders mit einer distalen Öffnung (111), einer proximalen Öffnung (112) und einem Dosierkanal (113) aufweist, der die distale und die proximale Öffnung (111, 112) verbindet, wobei zwischen dem Dosierkanal (113) und der distalen Öffnung (111) ein Einwegventil (115) angeordnet ist, wobei die proximale Öffnung (112) des Behälters (110) einen Einfüllkegel bildet, um das Befüllen des Dosierkanals (113) mit einer Pulverdosis zu erleichtern.

7. Vorrichtung nach Anspruch 6, wobei das Einwegventil (115) vom Typ mit geschlitzter Membran ist, das sich unter Wirkung eines vorbestimmten Drucks nur in eine Richtung öffnet.

8. Vorrichtung nach einem der vorstehenden Ansprüche, wobei der Ausgabekopf (300) ein Ausgabeelement (311) aufweist, das mit der Ausgabeöffnung (310) versehen ist.

9. Vorrichtung nach Anspruch 8, wobei das Ausgabeelement (311) eine Nadel aufweist.

10. Vorrichtung nach Anspruch 9, wobei die Nadl (311) gekrümmt oder abgewinkelt ist.

## Claims

1. Device for dispensing fluid product comprising a reservoir unit (100) connected on the one hand to an air expulsion system (200) and on the other hand to a dispensing head (300) provided with a dispensing orifice (310), wherein said air expulsion system comprises a pump (200) comprising an external body (210) which contains a pump body (220; 270) comprising a pumping chamber (225) and in which there is arranged a rod (230) acting as a piston, said rod (230) being fixed with respect to said external body (210), and said pump body (220; 270) being axially movable to slide around said rod (230) between a rest position and an actuation position, said pump body comprising a proximal hollow body (220) with respect to said dispensing orifice and a distal hollow body (270) with respect to said dispensing orifice fixed to one another, said proximal hollow body (220) comprising a proximal pump body part (221) with respect to said dispensing orifice and a distal pump body part (222) with respect to said dispensing orifice of which the inside diameter is greater than that of said proximal pump body part (221), said rod (230) comprising a proximal rod part (231) with respect to said dispensing orifice, a central rod part (232) and a distal rod part (233) with respect to said dispensing orifice, the outside diameter of said central rod part (232) being greater than the diameters of said proximal (231) and distal (233) rod parts, said central rod part (232) supporting a first seal (237) and a second seal (236), which, in rest position, collaborate in a sealed manner with said proximal pump body (221), and said distal rod part (233) supporting a third seal (235), which, during actuation, thus collaborates in a sealed manner with said distal hollow body (270) to isolate the said pumping chamber (225) from the atmosphere, the air contained in the said pumping chamber (225) being thus compressed and said compressed air being released when the said second seal (236) no longer collaborates in a sealed manner with said proximal pump body part (221), said proximal rod part (231) comprising a central passage (234) extending as far as said central rod part (232) beyond said first seal (237) and which opens laterally to the outside of said central rod part (232) between said first and second seals (237, 236), said distal hollow body (270) containing at least one spring (240, 241) collaborating on the one hand with said distal rod part (233) and on the other hand with said distal hollow body (270).

2. Device according to claim 1, wherein said proximal hollow body (220) and said distal hollow body (270) are fixed to one another, in particular by screwing, in a sealed manner with interposition of a body seal (260).

3. Device according to claim 1 or 2, wherein said distal rod part (233) slides in said distal hollow body (270), said third seal (235) performing the sealing therebetween during actuation.

4. Device according to any one of the preceding claims, wherein said distal hollow body (270) contains two springs (240, 241) arranged axially behind one another by being connected by a connecting member (245).

5. Device according to any one of the preceding claims, wherein an adjustment element (250), such an adjustment screw, is arranged in the distal hollow body (270) to form the contact with said spring (240), enabling to modify the actuation force exerted by the spring(s) on said rod (230) during the actuation.

6. Device according to any one of the preceding claims, wherein said reservoir unit (100) comprises a reservoir (110) having substantially the shape of a hollow cylinder, with a distal opening (111), a proximal opening (112) and a dosing passage (113) connecting said distal and proximal openings (111, 112), a one-directional valve (115) being arranged between said dosing passage (113) and distal opening (111), said proximal opening (112) of said reservoir (110) forming a filling cone to facilitate the filling of said dosing passage (113) with a dose of powder.

7. Device according to claim 6, wherein said one-directional valve (115) is of the split membrane type which only opens in one single direction under the effect of a predetermined pressure.

8. Device according to any one of the preceding claims, wherein said dispensing head (300) comprises a dispensing member (311) provided with said dispensing orifice (310).

9. Device according to claim 8, wherein said dispensing member (311) comprises a needle.

10. Device according to claim 9, wherein said needle (311) is curved or bent.
